# EUROPÄISCHE PATENTANMELDUNG

(11) **EP 3 839 031 A1**
(43) Veröffentlichungstag der Anmeldung: **23.06.2021**
(21) Anmeldenummer: 20214067.9
(22) Anmeldetag: 15.12.2020
(51) Int. Cl.: C12M 1/00, A01N 63/40, A61K 35/76, C12M 1/26

(54) **PHAGENKULTIVIERUNGSVORRICHTUNG, VERFAHREN ZUR PRÄPARATION VON PHAGEN UND FILTERVORRICHTUNG DAFÜR**

(30) Priorität: 16.12.2019 DE 102019134489
(71) Anmelder: Fachhochschule Südwestfalen, 58636 Iserlohn (DE)
(72) Erfinder: Hennes, Kilian, 58638 Iserlohn (DE)
(74) Vertreter: Cohausz Hannig Borkowski Wißgott

(57) **Zusammenfassung**

Die Erfindung betrifft eine Phagenkultivierungsvorrichtung umfassend ein zur äußeren Umgebung abgeschlossenes Fluidleitungssystem mit einem Eingangs-Port (1), an den ein Probengefäß (4) angeschlossen oder zumindest anschließbar ist, insbesondere so dass Phagen aus einer in das Probengefäß eingelegten/einlegbaren Probe (5) über den Eingangs-Port (1) in das Fluidleitungssystem überführbar sind,einem Ausgangs-Port (28), an dem ein Entnahmegefäß (27) abnehmbar befestigt oder zumindest befestigbar ist, insbesondere so dass Phagen, vorzugsweise aus den überführten Phagen durch Kultivierung multiplizierte Phagen, aus dem Fluidleitungssystem über den Ausgangs-Port entnehmbar sind, einer in einer Fluidleitung zwischen dem Eingangs-Port und dem Ausgangs-Port (28) angeordneten Filtervorrichtung (15) mit einer in Richtung zum Ausgangsport (28) für Phagen durchlässigen und für Bakterien undurchlässigen Filtermembran (19) wenigstens einem ein Nährmedium aufweisenden Nährmedienreservoir (3), wenigstens einem, insbesondere vor dessen Benutzung, geschlossenen, Wirtsbakterien aufweisenden Wirtsbakterienreservoir (6), das mit dem wenigstens einen Nährmedienreservoir (3) durch eine öffenbare Verbindungsleitung in Verbindung bringbar ist, insbesondere dessen Wirtsbakterien in gefriergetrockneter, lebensfähiger Form vorliegen, wobei das wenigstens eine Nährmedienreservoir (3) im Fluidleitungssystem betrachtet in der Flußrichtung vom Eingangs-Port (1) zum Ausgangs-Port vor der Filtervorrichtung (15) angeordnet ist, insbesondere so in das Fluidleitungssystem überführte Phagen vor der Filtervorrichtung (15) in dem wenigstens einen Nährmedienreservoir (3) mit den Wirtsbakterien wenigstens einmalig, vorzugsweise mehrmalig durch Kultivierung vermehrbar sind. Die Erfindung betrifft auf ein Verfahren zur Präparatiuon von Phagen und eine Tangentialflußfiltervorrichtung (15).

## Beschreibung

Die Erfindung betrifft eine Phagenkultivierungsvorrichtung. Die Erfindung betrifft weiterhin eine Filtervorrichtung für den Einsatz bei der Präparation kultivierter Phagen und ein Verfahren zur Kultivierung von Phagen.

Phagen, bzw. Bakteriophagen sind auf Bakterien als Wirte spezialisierte Viren, welche sich an die Bakterien anheften, ihr Erbgut in deren Zellen einschleusen, sich hierdurch im Zellinneren kopieren und durch die Kopien die Wirte zum Aufplatzen unter Freisetzung der Kopien bringen. Hierbei haben die Phagen eine hohe Spezifität, können daher also auch gut zur Bekämpfung bestimmter Bakterien zum Einsatz kommen. Im Stand der Technik ist somit auch die sogenannte Phagentherapie bekannt, die hauptsächlich in Georgien und der ehemaligen Sowjetunion zum Einsatz kommt, z.B. zur Bekämpfung multiresistenter Bakterien, gegen die ansonsten keine Antibiotika mehr helfen.

Im westeuropäischen Bereich scheitert der mögliche Einsatz von Phagen bislang mit wenigen Ausnahmen an den Hürden der benötigten langwierigen Zulassungsverfahren von für Therapiezwecke kultivierten Phagen für den Einsatz als Arzneimittel. Solche Hürden bestehen in den Ländern der ehemaligen Sowjetunion nicht. Aus diesen Ländern ist es somit aufgrund jahrzehntelanger klinischer Erfahrung bekannt Phagen spezifisch für ein Bakterium zu kultivieren und hiernach an dem Bakterium erkrankten Menschen als Präparat zur Therapie zu verabreichen. Die zur Multiplikation verwendeten Phagen stammen dabei häufig aus der Umwelt, bevorzugt aus Abwässern, sind damit voraussichtlich auch menschlichen Ursprunges, lassen sich aber nicht konkret einem Menschen zuordnen.

Es ist eine Aufgabe der Erfindung eine Vorrichtung und ein Verfahren bereit zu stellen, mit dem die Möglichkeit erschlossen wird, ein Präparat von autologen Phagen klinisch bereit zu stellen, also solche die nachweisbar auf einen ganz bestimmten Menschen als Ursprung zurückgehen und bevorzugt auch nur für den Einsatz an diesem einen ganz bestimmten Menschen bestimmt sind. Es ist eine Aufgabe der Erfindung durch Bereitstellung einer Vorrichtung als erfindungsgemäßes Medizinprodukt zu erschließen, dass Phagen als autologes Arzneimittel gemäß deutschem Arzneimittelgesetz und europäischen Gesetzen autolog hergestellt und auch ohne aufwändige Zulassungsverfahren am Menschen innerhalb kurzer Zeit zur Anwendung kommen können. Unabhängig von der vorgenannten Möglichkeit der Präparation autologer Phagen soll die Erfindung aber auch allgemein erschließen, Phagen in einem für Arztpraxen tauglichen Maßstab unter Einhaltung höchster Sicherheits- und Hygieneanforderungen präparieren zu können.

Diese Aufgabe wird dadurch gelöst, dass eine Phagenkultivierungsvorrichtung ein Fluidleitungssystem aufweist, insbesondere welches zur äußeren Umgebung während der gesamten Zeit der Kultivierung von Phagen abgeschlossen, bevorzugt hermetisch verschlossen ist, z.B. durch Luer-Lock-Konnektoren.

Ein ggfs. möglicher Kontakt von Phagen mit der Umgebung soll bevorzugt nur während der Entnahme einer Phagenprobe, z.B. von einem ganz bestimmten erkrankten Menschen und der Überführung der Phagen aus der Probe in die erfindungsgemäße Kultivierungsvorrichtung möglich sein. Während der gesamten Zeit der Kultivierung und der damit einhergehenden Vermehrung von Phagen auf der Basis der Phagenprobe ist jeglicher Austausch von Phagen oder auch Bakterien mit der Umgebung erfindungsgemäß durch die Geschlossenheit der Vorrichtung ausgeschlossen.

Eine solche erfindungsgemäße Vorrichtung weist einen Eingangs-Port auf, an den ein Probengefäß angeschlossen ist oder zumindest anschließbar ist.
Es wird so sichergestellt, dass Phagen aus einer in das Probengefäß eingelegten/einlegbaren Probe über den Eingangs-Port in das Fluidleitungssystem überführbar sind.

Wenn ein Probengefäß mit darin befindlicher Probe von Phagen an die Vorrichtung anschließbar ist, kann die Erfindung bevorzugt vorsehen, dass der Eingangs-Port bis zum Ansetzen des Probengefäßes an diesen geschlossen ist, insbesondere erst durch das Ansetzen des Probengefäßes an den Eingangs-Port geöffnet wird.

Insbesondere bei einem an der Vorrichtung befindlichen Probengefäß, in das durch Öffnen des Probengefäßes eine Probe von Phagen eingelegt werden kann, aber auch bei einem anschließbaren Probengefäß kann es die Erfindung vorsehen, dass die Verbindung zwischen dem Inneren des Probengefäßes mit der Probe und dem Inneren der Vorrichtung erst erstellt wird durch Aufbau eines Unterdruckes im Probengefäß, z.B. durch Vergrößerung von dessen inneren Volumen. Dies kann beispielsweise erst erfolgen, wenn ein zum Probeneinlegen öffenbares Probengefäß geschlossen ist. Die Erfindung kann auch vorsehen, dass ein mit der Vorrichtung verbindbares Probengefäß nach der Erstellung der Verbindung nicht wieder abgenommen werden kann, z.B. durch eine verriegelnde Verbindung. Eine nicht wieder öffenbare Verriegelung kann auch bei einem zum Probeneinlegen öffenbaren / geöffneten Probengefäß vorgesehen sein. Insbesondere durch diese Maßnahmen kann sichergestellt werden, dass die Vorrichtung während einer gesamten vorgesehenen Kultivierungszeit geschlossen ist und bleibt. Dies garantiert, dass sämtliche durch die Kultivierung multiplizierten Phagen ausschließlich auf die Phagen der Probe zurückzuführen sind.

Die Phagen in der Probe können bevorzugt von einem ganz bestimmten Menschen stammen, der an einem multiresistenten Keim (antibiotika-resistenten Bakterium) erkrankt ist. In diesem Fall handelt es sich um sogenannte autologe Phagen. Z.B. können die Phagen bereit gestellt werden durch einen Abstrich von der Nasenschleimhaut des bestimmten Menschen. Die durch Kultivierung multiplizierten Phagen sind somit in diesem Fall auch allesamt autologe Phagen. Grundsätzlich können im Rahmen der Erfindung aber auch Phagen von beliebiger Herkunft kultiviert werden.

Die erfindungsgemäße Vorrichtung umfasst weiterhin einen Ausgangs-Port, an dem ein Entnahmegefäß abnehmbar befestigt oder zumindest befestigbar ist, insbesondere so dass Phagen, vorzugsweise aus den von der Probe überführten Phagen durch Kultivierung multiplizierte Phagen, aus dem Fluidleitungssystem über den Ausgangs-Port entnehmbar sind.

Die Erfindung weist weiterhin eine in einer Fluidleitung zwischen dem Eingangs-Port und dem Ausgangs-Port angeordnete Filtervorrichtung mit einer in Richtung zum Ausgangs-Port für Phagen durchlässigen und für Bakterien undurchlässigen Filtermembran auf. So können die in der Vorrichtung multiplizierten Phagen von Bakterien getrennt werden und es kann sichergestellt werden, dass keine Infektionen eines mit den multiplizierten Phagen zu behandelnden Menschen oder auch anderen Lebewesens mit Bakterien aus der Vorrichtung erfolgen können.

Die Vorrichtung der Erfindung weist wenigstens ein Nährmedienreservoir mit einen Nährmedium auf und wenigstens ein, insbesondere vor dessen Benutzung, geschlossenes, Wirtsbakterien aufweisendes Wirtsbakterienreservoir, das mit dem wenigstens einen Nährmedienreservoir durch eine öffenbare Verbindungsleitung in Verbindung bringbar ist. Die Wirtsbakterien können in dem Wirtsbakterienreservoir in gefriergetrockneter, lebensfähiger Form oder in einer sonstigen Form von Dauerstadien vorliegen.

Das Nährmedium ist dabei auf die Wirtsbakterien des Wirtsbakterienreservoirs abgestimmt, hält diese also während der Kultivierungsdauer am Leben.

Das Nährmedium ist vorzugsweise ein flüssiges Nährmedium umfassend Wasser und eine für die Wirtsbakterien verwertbare Energiequelle, beispielsweise organische Verbindungen oder schwefelhaltige Verbindungen, sowie von den Wirtsbakterien benötigte Nährstoffe (z.B. organische oder anorganische Kohlenstoff-, Stickstoff-, Schwefel- und Phosphat-Quellen sowie andere essentielle Nährstoffe). Als Nährstoffe können z.B. Kohlenhydrate ("Zucker"), Proteinhydrolysate (Peptone) und gegebenenfalls Fettsäuren vorgesehen sein.

Durch die Trennung zwischen dem Nährmedienreservoir und dem Wirtsbakterienreservoir kann weiterhin erzielt werden, dass eine erfindungsgemäße Vorrichtung nahezu beliebig lange bis zu einem Einsatz gelagert werden kann.

Dies erschließt auch die Möglichkeit, Sets von mehreren erfindungsgemäßen Vorrichtungen bereit zu stellen, bei denen verschiedene Vorrichtungen mit jeweils unterschiedlichen Wirtsbakterien, insbesondere mit verschiedenen nichtresistenten Wirtsbakterien vom Typ eines klinisch relevanten antibiotikaresistenten (nachfolgend "resistent" genannten) Bakterienstamms ausgeführt sind. So kann nach Analyse eines resistenten Bakterienstammes bei einem erkrankten Menschen aus in Lagern befindlichen Vorrichtungen eine mit dem dazu passenden Wirtsbakterienstamm ausgewählt werden.

Bevorzugt erst im Einsatzmoment der Vorrichtung werden die Reservoirs verbunden.

Verfahrensgemäß wird dabei das Nährmedium in dem Nährmedienreservoir mit Wirtsbakterien, vorzugsweise nicht-resistenten Wirtsbakterien aus dem Wirtsbakterienreservoir versetzt. Diese Wirtsbakterien sind vorzugsweise als Typstamm eines vorbestimmten Typs von multiresistenten Bakterien gewählt.

Es kann z.B. vorgesehen sein, bei einem an einem multiresistenten Bakterium erkrankten Menschen das Bakterium zu analysieren und solche Wirtsbakterien zu wählen, die nicht-resistenter Art und von demselben Bakterientypenstamm sind. Solche Bakterien können z.B. aus einer Bakteriensammlung bezogen werden. Die vorzugsweise nicht-resistenten Wirtsbakterien werden im Nährmedium kultiviert, insbesondere hierbei aus einem gefriergetrockneten Zustand oder einem sonstigen Dauerstadium reaktiviert.

Die Verbindung zwischen Nährmedienreservoir und Wirtsbakterienreservoir kann z.B. durch ein Ventil erfolgen. Beispielsweise kann das Nährmedium, welches bevorzugt in flüssiger Form vorliegt durch die Verbindung in das Wirtsbakterienreservoir überführt und mit den Wirtsbakterien vermischt werden, wonach das mit Bakterien vermische Nährmedium wieder in das Nährmedienreservoir zurückgeführt wird. Z.B. kann dies dadurch erfolgen, dass die Reservoirs gedrückt werden, z.B. manuell. Dies ist insbesondere möglich, wenn die Reservoirs als flexible Beutel (z.B. aus PVC oder anderen für Blutbeutel geeigneten Kunststoffen) ausgeführt sind.

Das wenigstens eine Nährmedienreservoir ist erfindungsgemäß im Fluidleitungssystem betrachtet in der Flußrichtung vom Eingangs-Port zum Ausgangs-Port vor der Filtervorrichtung angeordnet.

Es sind so in das Fluidleitungssystem überführte Phagen vor der Filtervorrichtung in dem wenigstens einen Nährmedienreservoir mit den Wirtsbakterien wenigstens einmalig, vorzugsweise mehrmalig durch Kultivierung vermehrbar. Nach der abgeschlossenen einmaligen oder auch einer mehrmaligen Kultivierung können dann die multiplizierten Phagen durch die Filtervorrichtung hindurch zum Ausgangs-Port transportiert werden und gelangen dort in das Auffanggefäß.

Die Erfindung sieht es vor, dass das mit Wirtsbakterien versetzte Nährmedium mit Phagen, insbesondere mit autologen Phagen von einer Probe in dem an das Fluidleitungssystem angeschlossenen Probengefäß versetzt wird, welche die vorzugsweise zuvor durch Analyse bestimmten multiresistenten Bakterien abtöten können, insbesondere wofür das Nährmedium zeitweise in ein die Probe aufweisendes Probengefäß überführt und nach Kontaktierung der Probe in das Nährmedienreservoir zurückgeführt wird.

Bei der zuvor genannten Probengewinnung am erkrankten Menschen, z.B. an dessen Nasenschleimhaut wird davon ausgegangen, dass der erkrankte Mensch selbst mit einer hohen Wahrscheinlichkeit bereits Phagen in sich trägt, die gegen dessen multiresistente Bakterien abtötend wirken, jedoch über den Körper des Menschen nicht den Weg zu einem betroffenen Organ finden.

Um Phagen für die Kultivierung in die erfindungsgemäße Vorrichtung zu überführen ist es bevorzugt vorsehen, dass der Eingangs-Port, insbesondere das daran angeschlossene Probengefäß mit dem wenigstens einem Nährmedienreservoir zumindest zeitweise in Fluid-Verbindung bringbar ist. Dies kann z.B. mittels eines Ventils, vorzugsweise mittels eines 3-Wege-Ventils erfolgen, das im Fluidleitungssystem zwischen dem Eingangs-Port und dem Nährmedienreservoir angeordnet ist.

Ein Dreiwege-Ventil hat hier den Vorteil, dass eine Verbindung zwischen dem Probengefäß am Eingangs-Port nicht nur zum genannten Nährmedienreservoir sondern auch wahlweise zu anderen Elementen der Vorrichtung geschaltet werden kann, z.B. zu einer Leitung mit der das mit Bakterien und Phagen versetzte Nährmedium zur Filtervorrichtung oder auch weiteren Nährmedienreservoirs geleitet werden kann. Der Filter bzw. die Filtermembran der Filtervorrichtung kann bevorzugt einen durchschnittlichen Porendurchmesser von kleiner gleich 0,45 µm, weiter bevorzugt kleiner gleich 0,2 µm, noch weiter bevorzugt kleiner gleich 0,1 µm aufweisen.

Bevorzugt kann es vorgesehen sein, dass das Probengefäß und das wenigstens eine Nährmedienreservoir in seinem Volumen jeweils änderbar ist, insbesondere das Probengefäß als Spritze, oder als nach dem Aspirationsprinzip oder Unterdruckprinzip arbeitendes Blutentnahmeröhrchen ausgebildet ist, und/oder das Nährmedienreservoir als flexibler Beutel ausgebildet ist, so dass durch eine vorzugsweise manuell ausführbare Volumenänderung das Nährmedium, insbesondere das bereits mit Wirtsbakterien versetzte Nährmedium, aus dem wenigstens einen Nährmedienreservoir zumindest zeitweise in das Probengefäß beförderbar und in das dasselbe oder ein anderes Nährmedienreservoir zurückbeförderbar ist.

Für eine alleinige oder auch eine Vorkultivierung kann das hierdurch aus der Probe mit den Phagen versetzte Nährmedium in dasselbe Nährmedienreservoir zurückbefördert werden, aus dem es stammte.

Zur Kultivierung kann die gesamte Vorrichtung (z.B. in einem Brutschrank) eine vorbestimmte Zeit (z.B. 10 Stunden) unter einer vorbestimmten Temperatur (z.B. 37 Grad Celsius) inkubiert werden, wodurch eine Multiplikation der Phagen unter Lyse der Wirtsbakterien erfolgt, wobei durch Lyse der Bakterien des gewählten Typstamms genau solche Phagen vermehrt werden, welche die vorbestimmten multiresistenten Bakterien abtöten. Vorzugweise werden aufgrund der hohen Spezifität der Phagen evtl. andere aus der Probe stammende Phagen nicht mit vermehrt.

Allgemein werden die im Fluidleitungssystem nach wenigstens einmaliger Kultivierung gewonnenen Phagen durch Filtration, vorzugsweise Tangentialflußfiltration, von Bakterien getrennt (z.B. in einer nachfolgend noch näher beschriebenen Einwegfiltrationskammer, insbesondere welche im Blisterverfahren hergestellt wurde). Die durch Filtration getrennten Phagen werden in ein am Fluidleitungssystem angeschlossenes Auffanggefäß überführt und vorzugsweise mit dem Auffanggefäß vom Fluidleitungssystem als einsetzbares, vorzugsweise autologes Präparat abgenommen.

Die Erfindung sieht es zumindest vor, die von der Probe stammenden Phagen zumindest einmalig zu kultivieren und hierbei zu selektionieren und zu vermehren. Die Anzahl der hierdurch gewonnenen Phagen kann evtl. nicht ausreichend sein.

Die Erfindung kann somit auch in bevorzugter Ausführung vorsehen, die Kultivierung der Phagen wenigstens einmalig zu wiederholen, insbesondere mit einem neuen Ansatz von Nährmedium und einem neuen Ansatz von denselben Wirtsbakterien, die in der erfindungsgemäßen Vorrichtung in weiteren Reservoirs vorliegen.

In bevorzugter Ausführung können betrachtet in der Flußrichtung vom Eingangs-Port zum Ausgangs-Port vor der Filtervorrichtung mehrere Nährmedienreservoirs angeordnet sind, die in der Flußrichtung aufeinander folgend an eine zur Filtervorrichtung führenden Verbindungsleitung angeschlossen sind, insbesondere jeweils über ein umschaltbares 3-Wegeventil, vorzugsweise so dass durch eine Volumenänderung des Probengefäßes oder eines Nährmedienreservoirs das Nährmedium aus einem der Nährmedienreservoirs entnehmbar und in ein in Flußrichtung nachfolgendes Nährmedienreservoir überführbar ist. So kann das, vorzugsweise bereits mit Wirtsbakterien versehene Nährmedium eines nachfolgenden Reservoirs mit den kultivierten Phagen eines vorausgehenden Reservoirs angeimpft werden.

Bevorzugt ist jedem von mehreren Nährmedienreservoirs jeweils ein eigener Wirtsbakterienvorrat zugeordnet, insbesondere der direkt am zugeordneten Nährmedienreservoir durch eine geschlossene, öffenbare Verbindungsleitung nur mit dem Nährmedium des zugeordneten Nährmedienreservoirs in Verbindung bringbar ist. Die Wirtsbakterien aller Wirtsbakterienreservoirs einer so weitergebildeten Vorrichtung sind alle von demselben Typstamm, insbesondere sind die Wirtsbakterien aller Wirtsbakterienreservoirs die gleichen .

Weiter bevorzugt ist zwischen zwei in Flußrichtung aufeinander folgenden Nährmedienreservoirs ein Sterilfilter angeordnet.

Unter einem Sterilfilter wird ein solcher Filter verstanden, der geeignet ist Bakterien und Bakterienfragmente zurückzuhalten. In der hier beschriebenen Anwendung können hingegen Phagen den Sterilfilter passieren. Die Filtermembran eines Sterilfilters kann bevorzugt einen durchschnittlichen Porendurchmesser von kleiner gleich 0,45 µm, weiter bevorzugt kleiner gleich 0,2 µm, noch weiter bevorzugt kleiner gleich 0,1 µm aufweisen.

So können zur wenigstens einmalig wiederholten Kultivierung vor der Filtration Phagen mit dem Nährmedium aus einem von mehreren Nährmedienreservoirs, vorzugsweise über einen Sterilfilter, in das Nährmedium eines weiteren Nährmedienreservoir überführt werden, welches zuvor mit Wirtsbakterien desselben gewählten Typstamms versetzt wurde. Hierdurch erfolgt eine weitere Kultivierung und demnach eine weitere Vermehrung. Eine zweifache Kultivierung ist erfindungsgemäß bevorzugt, es kann jedoch auch vorgesehen sein, den genannten Kultivierungsschritt noch öfter durchzuführen.

Schlußendlich wird am Ende der Kultivierung eine Abtrennung der multiplizierten, bevorzugt autologen Phagen von den Bakterien im Nährmedium vorgenommen durch die eingangs genannte Filtervorrichtung.

Besonders bevorzugt ist die Filtervorrichtung als Tangentialflußfiltervorrichtung ausgebildet, bei welcher die Filtermembran auf einer der beiden Membranseiten tangential von dem Nährmedium, insbesondere reversierend überströmt ist.

Hierdurch wird eine feste Ansammlung von Bakterien an der Membran verhindert oder zumindest verzögert und die Filtrationseffizienz erhöht.

Die bevorzugt als Tangentialflußfiltervorrichtung ausgebildete Filtervorrichtung kann vorzugsweise eine erste und eine zweite im Volumen änderbare, vorzugsweise manuell änderbare Kavität aufweisen, die über wenigstens einen Kanal, bevorzugt mehrere parallele Kanäle, miteinander verbunden sind, dessen Wandung zumindest bereichsweise durch eine Filtermembran ausgebildet ist, die an ihrer zum Kanalinneren weisenden ersten Seite von zwischen der ersten und zweiten Kavität hin- und herpumpbaren Nährmedium tangential überströmbar ist, und an ihrer vom Kanalinneren abgewandten zweiten Seite an eine dritte Kavität angrenzt, wobei in die erste Kavität ein Eingangskanal und in die dritte Kavität ein Ausgangskanal mündet.

Der in die erste Kavität mündende Eingangskanal kann das Ende der Fluidleitung des erfindungsgemäßen Systems bilden, durch den nach der Kultivierung die Phagen mit dem Nährmedium der Filtervorrichtung zugeführt werden. Dieses Zuführen kann z.B. in einer Ausführung rein durch Schwerkraft erfolgen, oder auch durch Volumenverkleinerung desjenigen Nährmedienreservoir, in dem die letzte Kultivierung oder ggfs. auch die einzige Kultivierung stattgefunden hat. Der Ausgangskanal der Filtervorrichtung führt in Richtung zum Auffanggefäß der Vorrichtung, z.B. direkt oder auch mittelbar über ein fluidförderndes Element.

Unabhängig von der konkreten Ausgestaltung der Filtervorrichtung ist die Membran vorzugsweise so ausgelegt, dass ausschließlich nur Phagen durch die Membran hindurchtreten können, hingegen Bakterien und deren durch Lyse entstandenen Reste durch die Membran aufgehalten werden. Die Filtermembran der Filtervorrichtung kann bevorzugt einen durchschnittlichen Porendurchmesser von kleiner gleich 0,45 µm, weiter bevorzugt kleiner gleich 0,2 µm, noch weiter bevorzugt kleiner gleich 0,1 µm aufweisen.

Z.B. kann die Erfindung vorsehen, dass die erste und zweite Kavität abwechselnd in ihren jeweiligen Volumen reduziert wird, z.B. durch manuelles Drücken auf die flexible Kavität (z.B. aus Polypropylen oder einem anderen Kunststoff), wodurch ein reversierender Fluidfluß des Nährmediums mit den multiplizierten Phagen erzielt wird und sich nach Filtration die Phagen in der dritten Kavität anreichern, die bevorzugt bezogen auf die Flußrichtung zwischen der ersten und zweiten Kavität auf der anderen Membranseite angeordnet ist.

Es kann hier vorgesehen sein, dass die erste Kavität, zweite Kavität, der Eingangskanal, sowie der wenigstens eine die Kavitäten verbindende Kanal in eine erste Folie eingeformt sind, insbesondere als aus der Folienebene herausstehende Ausbauchung eingeformt sind und die dritte Kavität und der Ausgangskanal in eine zweite Folie eingeformt sind, insbesondere als aus der Folienebene herausstehende Ausbauchung eingeformt sind, wobei die erste und zweite Folie von gegenüberliegenden Seiten an den Oberflächen einer Filtermembran befestigt, insbesondere angeschweißt oder anlaminiert sind. Diese Folie kann aus dem genannten Polypropylen ausgebildet sein oder einem anderen Kunststoff.

Hierdurch ergibt sich eine durch die Flexibilität der Folien einfach manuell betätigbare Filtervorrichtung mit manueller Pumpfunktion. Die Erfindung kann es auch vorsehen, dass die Volumina der Kavitäten auch aktorisch komprimiert werden.

Aus Sicherheitsgründen kann es die Erfindung auch vorsehen, dass jede der beiden Folien von einer flexiblen Schale überdeckt ist, die an der jeweiligen Folie, insbesondere entlang des Randes der Filtermembran dicht befestigt ist, insbesondere durch Lamination / Verschweißung.

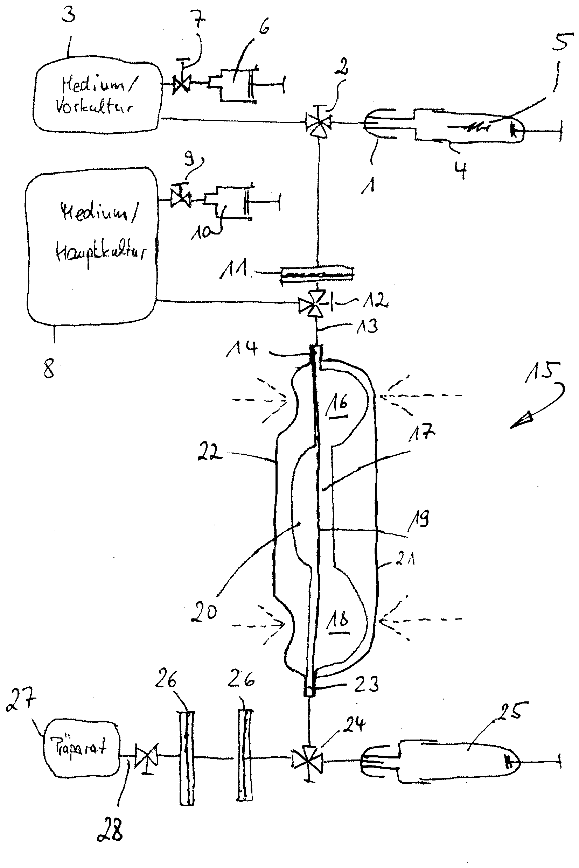

Eine Ausführung der Erfindung wird anhand der Figur 1 näher beschrieben.

Die Erfindung nutzt vorzugsweise ein zweistufiges Phagen-Anreicherungsverfahren mit nachgeschalteter Sterilfiltration und Abfüllung unter Nutzung der dargestellten Vorrichtung. Dabei umfasst die erfindungsgemäße in der Figur 1 dargestellte Vorrichtung ein System mit Fluidleitungen, Beuteln und Filtern.

Die Vorrichtung hat einen Eingangs-Port 1, der über ein Dreiwegeventil 2 mit einem Nährmedienreservoir 3 verbunden ist, an den als Probengefäß 4 z.B. eine sogenannte Monovette anschließbar ist, die eine Tupferspitze von einem Nasenabstrich eines an multiresistenten Bakterien erkrankten Patienten aufnimmt. Die Tuperspitze stellt eine Probe 5 mit Phagen dar, die der später zu behandelnde Patient selbst in sich trägt (z.B. als nicht körpereigenes Immunsystem).

Der Dreiwegehahn 2 ist über eine Leitung mit dem Nährmedien-Reservoir 3 verbunden, der z.B. durch einen Blutbeutel ausgebildet sein kann, hier für die Vorkultur der Phagen aus der Tupferspitze 5. Dieser Nährmedienbeutel 3 ist seinerseits mit einem Wirtbakterienreservoir 6 mit einem Wirtsbakterienvorrat, z.B. in einer Einwegspritze über ein Ventil 7 verbunden, in welchem gefriergetrocknete aber lebensfähige Wirtsbakterien vorgelegt sind. Diese Wirtsbakterien wurden als Typstamm desjenigen multiresistenten Keimes ausgewählt, an dem derjenige Patient leidet, von dem auch der Nasenabstrich auf der Tupferprobe genommen wurde.

Die Wirtsbakterien wurden z.B. zuvor von einer anerkannten Sammlung bezogen und weisen keinerlei Antibiotikaresistenzen auf. Beim Anschluss des Probengefäßes 4 an das Dreiwegeventil 2 wird zunächst das Ventil 7 zwischen dem Wirtsbakterienreservoir 6 und dem Nährmedienbeutel 3 geöffnet und durch Einsaugen des Nährmediums aus dem Beutel 3 werden die gefriergetrockneten Wirtsbakterien reaktiviert und in den Beutel 3 zurückgespült. Nach einer bevorzugt vorgenommenen Vorkultivierungsdauer von einigen Stunden bei geeigneter Bebrütungstemperatur (beispielsweise 37 °C) wird das Dreiwegeventil 2 geöffnet und die aktivierten Keime / Wirtsbakterien werden in das Probengefäß 4 gezogen durch Vergrößerung des Volumens von dem Probengefäß 4.

Dort kann es beim Umspülen der Tupferspitze 5 zur Adsorption von Phagen aus dem Nasenabstrich an die reaktivierten Wirtsbakterien kommen. Durch anschließende Bebrütung, z.B. über Nacht wird den in der Probe vorhandenen Phagen Gelegenheit gegeben, die daraufhin infizierte Bakterienkultur zu lysieren. Dabei werden nur die mit diesen Wirtsbakterien kompatiblen Phagen vermehrt.

Ebenfalls vorzugsweise über Nacht wird aus einem an die vorzugsweise als Einwegvorrichtung ausgebildete Vorrichtung der Erfindung angeschlossen weiteren Nährmedienreservoir 8 durch ein dazu geöffnetes Ventil 9 das Nährmedium in ein zweites nur an diesen Beutel 8 angeschlossenes, z.B. durch eine Spritze ausgebildetes Wirtsbakterienreservoir 10 angesaugt. Die in diesem Wirtsbakterienreservoir 10 vorgelegten gefriergetrockneten Wirtsbakterien des selben Typstammes wie zuvor beschrieben werden durch das Nährmedium Medium reaktiviert und anschließend in das als Hauptkulturbeutel ausgebildete Nährmedienreservoir 8 zurückgespritzt. Dort können die Bakterien sich z.B. über Nacht vermehren, während in der Vorkultur die gleichen Bakterien lysiert werden, sofern im Nasenabstrich lytische Phagen vorhanden waren.

Anschließend wird die infizierte Wirtsbakterienkultur aus dem Nährmedienreservoir 3 der Vorkultur durch das Dreiwegeventil 2 in das Probengefäß, z.B. die Monovette aufgezogen und nach Umschaltung des Ventils oder Abklemmen des Vorkulturbeutels 3 durch einen ebenfalls an das Dreiwegeventil 2 vorzugsweise angeschlossenen Sterilfilter 11 unter Ausdrücken aus dem Probengefäß 4 gefiltert. Dabei passieren die vorzugsweise über Nacht in der Vorkultur produzierten Phagen den Sterilfilter 11 und gelangen durch einen entsprechend geöffnetes zweiten Dreiwegeventil 12 hinter dem Sterilfilter 11 in die z.B. über Nacht vermehrte Hauptkultur im Beutel des weiteren Nährmedienreservoirs 8.

Die vorzugsweise über Nacht von der Vorkultur vermehrten Phagen können nun im Hauptkultur-Beutel 8 die dort ebenfalls vermehrten Wirtsbakterien vom nicht-antibiotikaresistenten Typstamm infizieren und während der Inkubation z.B. im Verlauf einer zweiten Nacht durch Lyse der Wirtsbakterien deutlich stärker vermehrt werden.

Das entsprechende Lysat mit Phagen wird anschließend nach Umstellen des zweiten Dreiwegeventils 12 z.B. mittels Gravitation über einen Zulauf 13 und den Eingangskanal 14 des Tangentialflußfilters 15 in diesen eingeleitet. Dort füllt das Lysat zumindest die erste Kavität 16, die durch wenigstens einen, vorzugsweise mehrere Kanäle 17 mit der zweiten Kavität 18 verbunden ist. Zumindest der Kanal ist über eine Filtermembran 19, also durch die Poren der Filtermembran hindurch mit einer dritten Kavität 20 verbunden. Im Filter 15 wird durch wechselseitiges Eindrücken der zwei flexiblen, retentat-seitiger Kavitäten 16 und 17 ein starker Tangentialfluss in dem wenigstens einen Kanal 17 über der Filtrationsmembran 19 erzeugt. Durch zwei aufgesiegelte umhüllende Kunststoffschalen 21 und 22 ist sichergestellt, dass auch im Falle des Berstens einer der
Kavitäten 16, 18 oder 20 keine Wirtsbakterien aus der Vorrichtung austreten können.

Aufgrund des Filtrationsprinzips der Tangentialflussfiltration wird eine effiziente Abtrennung der vermehrten Phagen von den vorzugsweise über Nacht durch Lyse entstandenen Zelltrümmern und den noch intakten Wirtsbakterien erreicht. Das Permeat wird in der permeatseitigen Kavität 20 des Tangentialflussfilters 15 gesammelt und anschließend über den Ausgangskanal 23 durch ein drittes Dreiwegeventil 24 in einer vorzugsweise vorgesehenen Spritze 25 aufgezogen.

Nach Umstellen des dritten Dreiwegeventils 24 wird das Permeat vorzugsweise durch zwei in Reihe gesteckte Sterilfilter 26 nach dem Dreiwegeventil 24 mittels Spritzendruck gepresst. Dieses Phagen-haltige Sterilfiltrat wird in einem angeschlossenen Auffanggefäß z.B. einem Präparatbeutel 27 aufgefangen und nach Verschließen des Ventils 24 von der erfindungsgemäßen Vorrichtung, vorzugsweise Einwegvorrichtung abgetrennt. Der Präparatbeutel 27 ist dafür abnehmbar an einem Ausgangs-Port der Vorrichtung befestigt.

Der Eingangs-Port 1 und der Ausgangs-Port 28 können z.B. durch Luer-Lock-Konnektoren ausgebildet sein.

Das in diesem ersten Anwendungsbeispiel hergestellte vorzugsweise autologe Phagenpräparat kann als patientenspezifische Zubereitung für neuartige medizinische Therapien, wie die Phagentherapie, eingesetzt werden.

In einem zweiten Anwendungsbeispiel werden nach demselben Prinzip Phagenpräparate aus Umweltproben mit Typstämmen multiresistenter Keime der Massentierhaltung hergestellt und als Futtermittelergänzung gegen die Verbreitung der multiresistenten Keime der Tiernahrung beigemischt.

In einem dritten bevorzugten Anwendungsbeispiel werden nach demselben Prinzip hergestellte Phagenpräparate zur Bekämpfung bakterieller Pflanzenkrankheiten auf den befallenen Plantagen versprüht.

In einem vierten bevorzugten Anwendungsbeispiel werden nach demselben Prinzip hergestellte Phagenpräparate zur Bekämpfung von Verderbnis- und Krankheitserregern auf handelsüblichen Lebensmitteln oder in Lebensm ittelproduktionsstätten versprüht.

## Patentansprüche

1. Phagenkultivierungsvorrichtung umfassend ein zur äußeren Umgebung abgeschlossenes Fluidleitungssystem mit
a. einem Eingangs-Port (1), an den ein Probengefäß (4) angeschlossen oder zumindest anschließbar ist, insbesondere so dass Phagen aus einer in das Probengefäß eingelegten/einlegbaren Probe (5) über den Eingangs-Port (1) in das Fluidleitungssystem überführbar sind,
b. einem Ausgangs-Port (28), an dem ein Entnahmegefäß (27) abnehmbar befestigt oder zumindest befestigbar ist, insbesondere so dass Phagen, vorzugsweise aus den überführten Phagen durch Kultivierung multiplizierte Phagen, aus dem Fluidleitungssystem über den Ausgangs-Port entnehmbar sind,
c. einer in einer Fluidleitung zwischen dem Eingangs-Port und dem Ausgangs-Port (28) angeordneten Filtervorrichtung (15) mit einer in Richtung zum Ausgangsport (28) für Phagen durchlässigen und für Bakterien undurchlässigen Filtermembran (19)
d. wenigstens einem ein Nährmedium aufweisenden Nährmedienreservoir (3),
e. wenigstens einem, insbesondere vor dessen Benutzung, geschlossenen, Wirtsbakterien aufweisenden Wirtsbakterienreservoir (6), das mit dem wenigstens einen Nährmedienreservoir (3) durch eine öffenbare Verbindungsleitung in Verbindung bringbar ist, insbesondere dessen Wirtsbakterien in gefriergetrockneter, lebensfähiger Form vorliegen,
wobei das wenigstens eine Nährmedienreservoir (3) im Fluidleitungssystem betrachtet in der Flußrichtung vom Eingangs-Port (1) zum Ausgangs-Port vor der Filtervorrichtung (15) angeordnet ist, insbesondere so in das Fluidleitungssystem überführte Phagen vor der Filtervorrichtung (15) in dem wenigstens einen Nährmedienreservoir (3) mit den Wirtsbakterien wenigstens einmalig, vorzugsweise mehrmalig durch Kultivierung vermehrbar sind.

2. Vorrichtung nach Anspruch 1, **dadurch gekennzeichnet, dass** der Eingangs-Port (1), insbesondere das daran angeschlossene Probengefäß (4) mit dem wenigstens einem Nährmedienreservoir (3) zumindest zeitweise in Fluid-Verbindung bringbar ist, insbesondere mittels eines Ventils (2), vorzugsweise 3-Wege-Ventils, das im Fluidleitungssystem zwischen dem Eingangs-Port (1)und dem Nährmedienreservoir (3) angeordnet ist.

3. Vorrichtung nach Anspruch 2, **dadurch gekennzeichnet, dass** das Probengefäß (4) und das wenigstens eine Nährmedienreservoir (3) in seinem Volumen jeweils änderbar ist, insbesondere das Probengefäß (4) als Spritze, oder als nach dem Aspirationsprinzip oder Unterdruckprinzip arbeitende Blutentnahmeröhrchen ausgebildet ist, und/oder das Nährmedienreservoir (3) als flexibler Beutel ausgebildet ist, so dass durch eine vorzugsweise manuell ausführbare Volumenänderung das Nährmedium, insbesondere das mit Wirtsbakterien versetzte Nährmedium, aus dem wenigstens einen Nährmedienreservoir (3) zumindest zeitweise in das Probengefäß (4) beförderbar und in das dasselbe (3) oder ein anderes Nährmedienreservoir (3, 8) zurückbeförderbar ist.

4. Vorrichtung nach einem der vorherigen Ansprüche, **dadurch gekennzeichnet, dass** betrachtet in der Flußrichtung vom Eingangs-Port (1) zum Ausgangs-Port vor der Filtervorrichtung (15) mehrere Nährmedienreservoirs (3, 8) angeordnet sind, die in der Flußrichtung aufeinander folgend an eine zur Filtervorrichtung führenden Verbindungsleitung (13) angeschlossen sind, insbesondere jeweils über ein umschaltbares 3-Wegeventil (2, 12), vorzugsweise so dass durch eine Volumenänderung des Probengefäßes (4) oder eines Nährmedienreservoirs (3, 8) das Nährmedium aus einem der Nährmedienreservoirs (3) entnehmbar und in ein in Flußrichtung nachfolgendes Nährmedienreservoir (8) überführbar ist.

5. Vorrichtung nach einem der vorherigen Ansprüche, **dadurch gekennzeichnet, dass** jedem von mehreren Nährmedienreservoirs (3, 8) jeweils ein eigener Wirtsbakterienvorrat (6, 10) zugeordnet ist, insbesondere der direkt am zugeordneten Nährmedienreservoir (3, 8) durch eine geschlossene, öffenbare Verbindungsleitung nur mit dem Nährmedium des zugeordneten Nährmedienreservoirs (3, 8) in Verbindung bringbar ist.

6. Vorrichtung nach Anspruch 4 oder 5, **dadurch gekennzeichnet, dass** zwischen zwei in Flußrichtung aufeinanderfolgenden Nährmedienreservoirs (3, 8) ein Sterilfilter (11) angeordnet ist.

7. Vorrichtung nach einem der vorherigen Ansprüche, **dadurch gekennzeichnet, dass** die Filtervorrichtung (15) als Tangentialflußfiltervorrichtung ausgebildet ist, bei welcher die Filtermembran (19) auf einer der beiden Membranseiten tangential von dem Nährmedium, insbesondere reversierend überströmt ist.

8. Set von mehreren Vorrichtungen nach einem der vorherigen Ansprüche, **dadurch gekennzeichnet, dass** es Vorrichtungen umfasst mit jeweils unterschiedlichen Wirtsbakterien, insbesondere mit verschiedenen nicht antibiotikaresistenten Wirtsbakterien vom Typ eines antibiotikaresistenten Bakterienstamms.

9. Tangentialflußfiltervorrichtung (15), insbesondere zum Einsatz in einer Vorrichtung nach einem der vorherigen Ansprüche, umfassend eine erste und eine zweite im Volumen änderbare, vorzugsweise manuell änderbare Kavität (16, 18), die über wenigstens einen Kanal (17), bevorzugt mehrere parallele Kanäle, miteinander verbunden sind, dessen Wandung zumindest bereichsweise durch eine Filtermembran (19) ausgebildet ist, die an ihrer zum Kanalinneren weisenden ersten Seite von zwischen der ersten und zweiten Kavität (16, 18) hin- und herpumpbaren Nährmedium tangential überströmbar ist, und an ihrer vom Kanalinneren abgewandten zweiten Seite an eine dritte Kavität (20) angrenzt, wobei in die erste Kavität (16) ein Eingangskanal (14) und in die dritte Kavität (20) ein Ausgangskanal (23) mündet.

10. Tangentialflußvorrichtung nach Anspruch 9, **dadurch gekennzeichnet, dass** die erste Kavität (16), zweite Kavität (18), der Eingangskanal (14), sowie der wenigstens eine die Kavitäten (16, 18) verbindende Kanal (17) in eine erste Folie eingeformt sind, insbesondere als aus der Folienebene herausstehende Ausbauchung eingeformt sind und die dritte Kavität (20) und der Ausgangskanal (23) in eine zweite Folie eingeformt sind, insbesondere als aus der Folienebene herausstehende Ausbauchung eingeformt sind, wobei die erste und zweite Folie von gegenüberliegenden Seiten an den Oberflächen einer Filtermembran (19) befestigt, insbesondere angeschweißt oder anlaminiert sind.

11. Tangentialflußvorrichtung nach Anspruch 10, **dadurch gekennzeichnet, dass** jeder der beiden Folien von einer flexiblen Schale (21, 22) überdeckt ist, die an der jeweiligen Folie dicht befestigt ist, insbesondere durch Lamination / Verschweißung.

12. Verfahren zur Präparation von Phagen für die Abtötung eines vorbestimmten Typs multi-antibiotikaresistenter Bakterien, **dadurch gekennzeichnet, dass** in einem zur äußeren Umwelt abgeschlossenen Fluidleitungssystem, insbesondere das als Phagenkultivationsvorrichtung nach einem der vorherigen Ansprüche ausgebildet ist, folgende Schritte durchgeführt werden:
a. ein Nährmedium in einem Nährmedienreservoir (3) wird mit nicht-antibiotikaresistenten Wirtsbakterien aus einem Wirtsbakterienreservoir (6) versetzt, die als Typstamm eines vorbestimmten Typs von multiantibiotikaresistenten Bakterien gewählt sind,
b. die nicht-antibiotikaresistenten Wirtsbakterien werden im Nährmedium kultiviert, insbesondere hierbei aus einem gefriergetrockeneten Zustand reaktiviert
c. das mit Wirtsbakterien versetzte Nährmedium wird mit Phagen, insbesondere mit autologen Phagen von einer Probe (5) in einem an das Fluidleitungssystem angeschlossenen Probengefäß (4) versetzt, welche die multiresistenten Bakterien abtöten, insbesondere wofür das Nährmedium zeitweise in ein die Probe (5) aufweisendes Probengefäß (4) überführt und nach Kontaktierung der Probe in das Nährmedienreservoir (3) zurückgeführt wird,
d. im Nährmedienreservoir (3) werden die von der Probe (5) überführten Phagen durch Kultivierung über eine vorbestimmte Zeit multipliziert, wobei durch Lyse der Bakterien des gewählten Typstamms solche Phagen vermehrt werden, welche die vorbestimmten multiantibiotikaresistenten Bakterien abtöten.
e. Die im Fluidleitungsystem nach wenigstens einmaliger Kultivierung gewonnenen Phagen werden durch Filtration, vorzugsweise Tangentialflußfiltration, von Bakterien getrennt
f. Die durch Filtration getrennten Phagen werden in ein am Fluidleitungssystem angeschlossenes Auffanggefäß (27) überführt, insbesondere hiernach mit dem Auffangsgefäß vom Fluidleitungssystem als einsetzbares, vorzugsweise autologes Präparat abgenommen.

13. Verfahren nach Anspruch 12, **dadurch gekennzeichnet, dass** zur wenigstens einmalig wiederholten Kultivierung vor der Filtration Phagen mit dem Nährmedium aus einem von mehreren Nährmedienreservoirs (3, 8), vorzugsweise über einen Sterilfilter (11), in das Nährmedium eines weiteren Nährmedienreservoir (8) überführt werden, welches zuvor mit Wirtsbakterien desselben gewählten Typstamms versetzt wurde.
